# EUROPEAN PATENT APPLICATION

(11) **EP 1 273 655 A1**
(43) Date of publication of application: **08.01.2003**
(21) Application number: 01917667.6
(22) Date of filing: 30.03.2001
(51) Int. Cl.: C12N 15/09, C12P 21/00, C12P 21/02

(54) **PROCESS FOR PRODUCING RECOMBINANT PROTEIN**

(30) Priority: 30.03.2000 JP 2000097891
(71) Applicant: Takeda Chemical Industries, Ltd., Osaka-shi, Osaka 541-0045 (JP)
(72) Inventor: YAMADA, Takao, Matsubara-shi, Osaka 580-0003 (JP); TSUJI, Isamu, Mino-shi, Osaka 562-0045 (JP); MATSUI, Hideki, Tsukuba-shi, Ibaraki 305-0044 (JP)
(74) Representative: Lewin, John Harvey
(86) International application number: JP0102712
(87) International publication number: WO01075095

(57) **Abstract**

The present invention relates to a process for producing a protein in the active form or salt thereof, which comprises extracting a protein, which has been expressed by genetic engineering in a prokaryotic host, with a solution containing about 0.1 mM to about 50 mM of a reducing agent having a reduction potential higher than -331 mV; and then refolding the protein in a solution containing a mercapto-free amino acid or its salt.

Using the production process of the present invention, a recombinant protein in the inactive form expressed in a prokaryotic cell can be efficiently made active and thus a biologically and pharmacologically active recombinant protein having the above-described effects can be prepared in a large amount.

## Description

### TECHNICAL FIELD

The present invention relates to a method of efficiently producing a recombinant protein in the biologically active form, which comprises denaturing and solubilizing a protein, which is expressed in a prokaryotic cell using genetic engineering, in the presence of a weak reducing agent (a reducing agent having a reduction potential of higher than -331 mV) at a low concentration (about 0.1 mM to about 50 mM, preferably about 0.1 mM to about 10 mM); and subjecting the protein to refolding operation.

### BACKGROUND ART

Multicellular organisms cleverly control proliferation and death of cells for retaining the homeostasis. In a process of ontogeny, a lot of cells are removed by cell death, and in a mature organism, cells constituting organs and tissues always keep balance of proliferation and death to maintain the function. Such cell death is considered as pre-determined death, called "Programmed Cell Death", and is known to occur via a process of Apoptosis, which is clearly morphologically distinguished from Necrosis, the cell death occurring unexpectedly due to physical and chemical causes.

So far, many physiological and pathological phenomena involving apoptosis have been clarified, and attempts to diagnose, prevent and treat various diseases by inducing or suppressing apoptosis of cells have been actively conducted (SCIENCE 267, 1456-1462, 1995). Apoptosis is one of life phenomena which are especially paid attention in this technical field.

Apoptosis is induced under various physiological conditions, and particularly, a Fas antigen (CD95, APO-1) attracts attention in recent years as a molecule to induce death of cells of immune system (SCIENCE 267, 1449-1456, 1995). The Fas antigen is a type I membrane protein belonging to the TNF (tumor necrosis factor) receptor family and having a molecular weight of 45 kDa, and induces cell death by binding with a Fas ligand. Expression of the Fas antigen is observed in various blood cells, and various tissues or cells thereof, such as liver, heart, small intestine and the like, while expression of a Fas ligand which is a type II membrane protein having a molecular weight of 40 kDa is limited to activated T lymphocytes, natural killer (NK) cells, macrophages, testicle, cornea and the like. Recently, analysis of genes in mice has clarified that a Fas antigen gene is a lpr structural gene itself, which has mutation in an autoimmune disease-developed mouse called lpr (lymphoproliferation) mouse, and that a Fas ligand has mutation in a gld (generalized-lymphoproliferative disease) mouse manifesting the same symptom as the lpr mouse. Also in case of humans, autoimmune disease-developed patients who have mutation in a Fas antigen gene are reported, and it is strongly suggested that dysfunction of the Fas/Fas ligand system causes an autoimmune disease (SCIENCE 268, 1347-1349, 1995).

Further, it has turned out that most of human Fas ligands are cut by matrix metalloproteinase to release soluble Fas ligands, and a possibility is also suggested that Fas ligands control immune response more widely, not only via cell-to-cell interaction (JOURNAL OF EXPERIMENTAL MEDICINE, 182, 1777-1783, 1995).

In addition to Fas ligands, TNF-α, Lymphotoxin-α (LT-α) and Lymphotoxin-β (LT-β) are reported to have an activity to induce apoptosis, among TNF family proteins having various biological activities (THE NEW ENGLAND JOURNAL OF MEDICINE, 334, 1717-1725, 1996).

Most recently, TL4 has been reported from Human Genome Science Company as a Fas ligand-like protein (Immunity 8, 21-30, 1998). TL4 is a protein constituted of 240 amino acid residues as shown in the Sequence Listing (SEQ ID NO: 1), and has a cytoplasmic tail composed of 37 residues and a transmembrane region composed of 22 residues at the N terminal. The receptor binding region (150 residues) on the C terminal side shows 25 to 35% homology with each of FasL, TNF-α, LT, CD40L, TRAIL and the like, and is expected to be released as a soluble ligand to manifest pharmacological action. Receptors for TL4 include HVEM (herpes virus entry mediator) and LTβR, both belonging to the TNFR family, and TR6/DcR3 present as a soluble decoy receptor.

It has recently been found that TL4 causes apoptosis in cancer cells expressing HVEM and LTβR, as the physiological action, and thus possibility of TL4 as an anticancer agent is expected (J. Clin. Invest. 102, 1142-1151, 1998; WO 98/-3648). Further, TL4 is also expected to have a function as an immunoregulator since TL4 promotes expression and secretion of IFNγ in activated PBL cells. Furthermore, most recently, an action of TL4 to enhance synthesis of DNA has been found in normal human hepatic parenchymal cells, and thus TL4 may also be useful as a hepatic function regulator (Japanese Patent Application No. 2000-014044).

In general, when a pharmacological effect of a physiologically active protein present only in small amount in an organism is investigated, it is common to express the protein in a recombinant form in large amount and purify it. In case of TL4, a Fas ligand-like protein, expression and secretion of active TL4 was also observed in an animal cell expression system using CHO cells and the like, or an insect cell expression system using Sf-9 cells and the like. However, these eucaryotic cells cannot be efficiently cultured, and further, the amount of expressed recombinant protein is not so large, and thus these methods accompany a lot of problems as the method of producing physiologically active proteins in industrial scale.

On the other hand, when prokaryotic cells such as *Escherichia coli* and the like are used as a host, high expression of a recombinant protein can be expected at a considerable probability. In this case, recombinant proteins produced in a large amount form an insoluble particle called inclusion body in *Escherichia coli,* and exist in a denatured and reduced form. Therefore, for obtaining a recombinant protein from an inclusion body, it is necessary to denature and extract the protein with denaturizing agent such as guanidine chloride and the like, and then to refold a polypeptide chain by an operation called refolding so as to give an active conformation. Actually, by using such a method, growth hormone, interleukin-2 and the like are produced from *Escherichia coli,* and formulated and sold as a pharmaceutical. However, this refolding operation shows a broad range of difficulty depending on properties of an individual protein. It is well known that particularly in case of proteins containing a lot of cysteine residues (therefore, forming a lot of disulfide bonds), the refolding operation is not so easy.

Actually, JP-A Nos. 4-218387 and 9-121886 disclose that DTT which is a strong reducing agent is used for extracting a target protein, although it is necessary to remove the reducing agent by dialysis, gel filtration and the like, before the refolding operation. Journal of Endocrinology (1997) 153, 139-150 discloses the addition of cysteine for extracting a target protein, while it is not clear whether this method is suitable as a method of producing physiological proteins in industrial scale or not.

TL4 which is a Fas ligand-like protein has two cysteine residues to form only one disulfide bond. However, when TL4 was expressed as a recombinant protein in *Escherichia coli*, it was impossible to efficiently obtain TL4 having an active conformation under these conventional refolding conditions.

### SUMMARY OF THE INVENTION

The present inventors intensively studied to provide an efficient activating method (renaturating method) utilize high productivity of a prokaryotic cell with the above-mentioned defects overcome. As a result, the inventors found that, in a method of activating a recombinant protein expressed in a prokaryotic cell, by combination of addition of a reducing agent at low concentration on extracting the protein and addition of an amino acid on refolding the protein led unexpectedly to a remarkable increase in the yield of the recombinant protein, using TL4, a Fas ligand-like protein as a specific example, and accomplished the present invention.

Thus, the present invention relates to a method of efficiently producing a recombinant protein or salt thereof, characterized by adding a reducing agent at lower concentration on extracting a protein, and adding an amino acid on refolding a protein, the protein which is expressed in a prokaryotic host cell by genetic engineering.

Specifically, the present invention provides:
(1) a method of producing a protein in the active form or salt thereof, comprising:
   expressing a protein in a prokaryotic host cell by genetic engineering;
   extracting the protein with a solution containing a reducing agent having a reduction potential of higher than -331 mV at a concentration of about 0.1 mM to about 50 mM; and
   refolding the protein in a solution containing a mercapto-free amino acid or salt thereof;
(2) the producing method described in the above (1), wherein refolding is conducted in a solution containing (i) a mercapto-free amino acid or salt thereof, and (ii) reduced glutathione and oxidized glutathione, cysteine and cystine, or cysteamine and cystamine;
(3) the producing method described in the above (1), wherein the protein is a Fas ligand-like protein;
(4) the producing method described in the above (3), wherein the Fas ligand-like protein is TL4;
(5) the producing method described in the above (1), wherein the reducing agent is a compound having a mercapto group;
(6) the producing method described in the above (5), wherein the compound having a mercapto group is 2-mercaptoethanol or cysteamine;
(7) the producing method described in the above (1), wherein the mercapto-free amino acid is arginine; and
(8) the producing method described in the above (1), comprising:
   expressing a protein in a prokaryotic host cell by genetic engineering;
   extracting and solubilizing the protein from the cell with a solution containing a reducing agent having a reduction potential of higher than -331 mV at a concentration of about 0.1 mM to about 50 mM, and a protein-denaturing agent; and
   diluting the extract with a refolding solution containing a mercapto-free amino acid or salt thereof to reach an ineffective concentration of the denaturing agent.

### BRIEF DESCRIPTION OF THE DRAWINGS

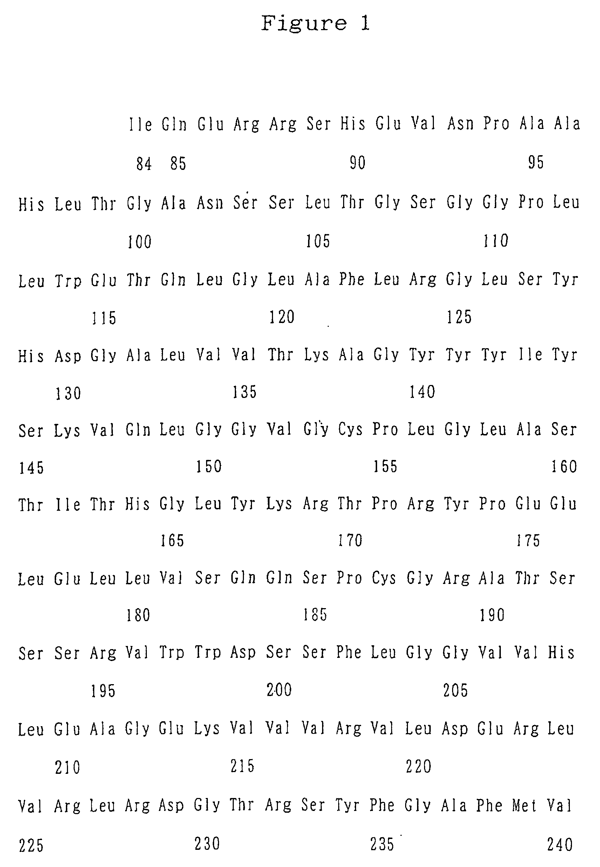

Fig. 1 shows an amino acid sequence of soluble human TL4 (Ile84-Val240).
Fig. 2 shows a construction of a plasmid pTCII-shTL4.
Fig. 3 shows behavior of a purified soluble human TL4 in SDS polyacrylamide gel electrophoresis. Lane 1 shows a molecular weight marker, and lane 2 shows a purified soluble human TL4. Multi Gel 15/25 (Daiichi Pure Chemicals Co., Ltd) was used as a gel, and Coomassie brilliant blue was used for staining.
Fig. 4 shows elution patterns of a purified soluble human TL4 on ion exchange HPLC and reverse phase HPLC.
Fig. 5 shows an effect of addition of 2-mercaptoethanol in extraction and an effect of addition of arginine in refolding, on the yield of soluble human TL4.
Fig. 6 shows a biological activity of soluble human TL4.
Fig. 7 shows an amino acid sequence of soluble mouse TL4 (Leu81-Val239).
Fig. 8 shows a construction of a plasmid pTCII-mTL4.
Fig. 9 shows behavior of a purified soluble mouse TL4 in SDS polyacrylamide gel electrophoresis. Lane 1 shows a molecular weight marker, and lane 2 shows a purified soluble mouse TL4. Multi Gel 15/25 (manufactured by Daiichi Pure Chemicals Co., Ltd) was used as a gel, and Coomassie brilliant blue was use for staining.
Fig. 10 shows elution patterns of a purified soluble mouse TL4 in ion exchange HPLC and reverse phase HPLC.
Fig. 11 shows an effect of addition of cysteamine in extraction and an effect of addition of arginine in refolding, on the yield of soluble mouse TL4.

### BEST MODES FOR CARRYING OUT THE INVENTION

The Fas ligand-like protein used in the examples of the present invention has the same activity as that of known Fas ligands, TNFα and the like, and it includes mammal-derived Fas ligand-like proteins and variants thereof having Met added to the N-terminal of said proteins. Among those, preferred are proteins comprising the amino acid sequence represented by SEQ ID NO: 1 or SEQ ID NO: 4 or partial peptides thereof, particularly, soluble human TL4 or soluble mouse TL4 comprising a partial amino acid sequence from 84-th (Ile) to 240-th (Val) from the N terminal of the amino acid sequence represented by SEQ ID NO: 1 or a partial amino acid sequence from 81-th (Leu) to 239-th (Val) from the N terminal of the amino acid sequence represented by SEQ ID NO: 4, and variants thereof which have Met added to the N-terminal of these proteins.

Further, mutains of soluble human TL4 or soluble mouse TL4 comprising a partial amino acid sequence from 84-th (Ile) to 240-th (Val) from the N terminal of the amino acid sequence represented by SEQ ID NO: 1 or a partial amino acid sequence from 81-th (Leu) to 239-th (Val) from the N terminal of the amino acid sequence represented by SEQ ID NO: 4, which have deletion of the N-terminal or C-terminal portion, and inversely, which have extention at the N-terminal or C-terminal, and which have substitution of a specific amino acid residue, may also be used, providing they have the same activity as that of said soluble human TL4 or soluble mouse TL4.

In the specification, the term "Fas ligand-like protein" is intended to include also proteins described in WO98/03648, WO97/34911, US Patent No. 5,874,240 and the like.

In the specification, the salts of proteins include pharmaceutically acceptable salts with inorganic acids such as hydrochloric acid, hydrobromic acid, nitric acid, sulfuric acid, phosphoric acid and the like, salts with organic acids such as acetic acid, phthalic acid, fumaric acid, tartaric acid, maleic acid, citric acid, succinic acid, methanesulfonic acid, p-toluenesulfonic acid and the like, alkali metal salts such as a sodium salt, potassium salt and the like, alkaline earth metal salts such as a calcium salt and the like, and an ammonium salt and the like, and hydrates thereof.

Prokaryotic cells used in the present invention includes Escherichia strains such as *Escherichia coli, Bacillus* strains such as *Bacillus subtilis, Serratia* strains such as *Serratia marcescens,* of which *Escherichia coli* and the like are preferable. Transformation, culturing and other treatments of these prokaryotic cells can be conducted according to conventional methods (for example, a method described in JP-A No. 3-204897, and the like), as well as the methods described below.

An expression vector containing cDNA encoding the Fas ligand-like protein used in the examples of the present invention can be produced, for example, by (i) isolating messenger RNAs (mRNAs) from a Fas ligand-like protein-producing cell, (ii) synthesizing single stranded cDNAs from the mRNAs, and then double stranded DNAs, (iii) inserting the complementary DNAs into a phage or plasmid, (iv) transforming a host with the resulting recombinant phages or plasmids, (v) culturing the transformants thus obtained, and then isolating a phage or plasmid containing the desired DNA from the transformant by a suitable method, for example, by hybridization with a DNA probe encoding a part of the Fas ligand-like protein or by an immunoassay method using an antibody, (vi) excising the desired cloned DNA from the recombinant DNA, and (vii) linking the cloned DNA or a part thereof downstream of a promoter in an expression vector.

The plasmid into which cDNA is integrated includes, for example, pBR322 [Gene, vol. 2, p. 95 (1977)], pBR325 [Gene, vol. 4, p. 121 (1978)], pUC12 [Gene, vol. 19, p. 259 (1982)], pUC13 [Gene, vol. 19, p. 259 (1982)], which are derived from *Escherichia coli,* pUB110 [Biochemical and Biophysical Research Communications, vol. 112, p. 678 (1983)] derived from *Bacillus subtilis,* and the like, and any other plasmids can also be used if they can be replicated and proliferated in a host. The phage vector into which cDNA is integrated includes, for example, λ gt11 [Young, R. and Davis, R., Proc. Natl. Acad. Sci., U.S.A., vol. 80, 1194 (1983)] and the like are listed, and any other vectors can also be used if they can be proliferated in a host.

The method for integration into a plasmid includes, for example, a method described in T. Maniatis et al., Molecular Cloning, Cold Spring Harbor Laboratory, p. 239 (1982). The method of integrating cDNA into a phage vector, for example, a method of Hyunh, T.V. et al. [DNA Cloning, A Practical Approach, vol. 1, p.49 (1985)].

The thus obtained plasmid is introduced into a suitable host, for example, an Escherichia strain, a Bacillus strain and the like.

Examples of the above-mentioned *Escherichia* strain include *Escherichia coli* K12DH1 [Proc. Natl. Acad. Sci. U.S.A., vol. 60, p. 160 (1968)], JM103 [Nucleic Acids Research, vol. 9, p. 309 (1981)], JA221 [Journal of Molecular Biology, vol. 120, p. 517 (1978)], HB101 [Journal of Molecular Biology, vol. 41, p. 459 (1969)], C600 [Genetics, vol. 39, p. 440 (1954)], MM294 [Nature, vol. 217, p. 1110 (1968)] and the like.

Examples of the above-mentioned Bacillus strain include, for example, Bacillus subtilis MI114 [Gene, vol. 24, 255 (1983)], 207-21 [Journal of Biochemistry, vol. 95, p. 87 (1984)] and the like.

The method of transforming a host with a plasmid includes, for example, a calcium chloride method or a calcium chloride/rubidium chloride method described in T. Maniatis et al., Molecular Cloning, Cold Spring Harbor Laboratory, p. 249 (1982), and the like.

When using a phage vector, it can be introduced into proliferated *Escherichia coli* using an in vitro packaging method.

The cDNA thus cloned encoding the Fas ligand-like protein can be, if necessary, subcloned into a plasmid, for example, pBR322, pUC12, pUC13, pUC18, pUC19, pUC118, pUC119, and the like.

The base sequence of thus obtained cDNA can be determined, for example, by the Maxam-Gilbert method [Maxam, A. M. and Gilbert, W., Proc. Natl. Acad. Sci., U.S.A., vol. 74, p. 560 (1977)] or the dideoxy method [Messing, J. et al., Nucleic Acids Research, vol. 9, p. 309 (1981)] to confirm the presence of cDNA of a Fas ligand-like protein via comparison with its already reported amino acid sequence.

As described above, the cDNA encoding a Fas ligand-like protein used in the examples of the present invention can be obtained.

The cDNA encoding a Fas ligand-like protein cloned as described above can be used as it is, or digested if necessary with a restriction enzyme or exonuclease, depending on the purpose.

Next, the expression vector can be obtained by excising a region to be expressed from the clone cDNA, and linking it downstream of a promoter in a vehicle (vector) suitable for the expression.

The cDNA may have ATG as a translation initiation codon at the 5' terminal, and TAA, TGA or TAG as a translation stop codon at the 3' terminal. The translation initiation codon and translation stop codon can also be added by using a suitable synthetic DNA adaptor. Further, a promoter is linked to the upstream of the DNA for expression.

For the vector, the above-mentioned *Escherichia coli*-derived plasmids (for example, pBR322, pBR325, pUC12, pCU13), Bacillus subtilis-derived plasmids (for example, pUB110, pTP5, pC194), and the like may be used.

The promoter used in the present invention may be any promoter which is suitable for expression of a gene in a corresponding host.

When a host for transformation is Escherichia strains, T7 promoter, trp promoter, lac promoter, recA promoter, λ PL promoter, lpp promoter and the like are preferable, and when a host for transformation is Bacillus strains, SPO1 promoter, SPO2 promoter, penP promoter and the like are preferable. It is particularly preferable that the host is an Escherichia strain and the promoter is T7 promoter, trp promoter or λ PL promoter.

Use of an enhancer is also effective for expression.

A transformant of a prokaryotic cell is produced by using the vector thus constituted containing cDNA encoding a Fas ligand-like protein.

Transformation of the above-mentioned Escherichia strain is conducted according to methods described, for example, in Proc. Natl. Acad. Sci. USA, vol. 69, p. 2110 (1972), Gene, vol. 17, p. 107 (1982) and the like.

Transformation of the Bacillus strain is conducted according to a method described, for example, in Molecular & General Genetics, vol. 168, p. 111 (1979), and the like.

In this way, a transformant of a prokaryotic cell, transformed with the expression vector containing cDNA encoding a Fas ligand-like protein, can be obtained. When an Escherichia strain is used as a host and T7 promoter is used as a promoter, a T7 lysozyme expression plasmid may also co-exist in addition to the expression vector containing cDNA encoding a Fas ligand-like protein, for the purpose of improving the expression efficiency of T7 promoter.

When an Escherichia or Bacillus transformant is cultured, the medium used for culturing is suitably a liquid medium, and it contains carbon sources, nitrogen sources, inorganic substances and other substances necessary for growth of the transformant. The carbon source includes, for example, glucose, dextrin, soluble starch, sucrose and the like, the nitrogen source includes inorganic or organic substances, such as ammonium salts, nitric acid salts, corn steep liquor, peptone, casein, meat extract, soybean cake, potato extracted and the like, and the inorganic substance includes, for example, calcium chloride, sodium dihydrogen phosphate, magnesium chloride and the like. Yeast extract, vitamins, growth promoting factor and the like may also be added. pH of the medium is preferably about 5 to 8.

The preferred medium for culturing an Escherichia strain is, for example, an M9 medium containing glucose and casamino acid [Miller, Journal of Experiments in Molecular Genetics, pp. 431-433, Cold Spring Harbor Laboratory, New York 1972], an LB medium and the like. For increasing efficiency of a promoter, if necessary, agents such as isopropyl-β-D-thiogalactopyranoside (IPTG) and 3β-indolylacrylic acid may be added.

When the host is an Escherichia strain, culturing is usually conducted at about 15 to 43°C for about 3 to 24 hours, and if necessary, aeration or stirring can also be added.

When the host is a Bacillus strain, culturing is usually conducted at about 30 to 40°C for about 6 to 24 hours, and if necessary, aeration or stirring can also be added.

When a recombinant protein forms an inclusion body in a prokaryotic host cell, a recombinant protein can be extracted by, after culturing, collecting the bacterium by a method such as centrifugal separation and the like, then, crushing cells, and solubilizing the inclusion body using a denaturing agent.

Crushing of cells can be carried out by an ordinary method, for example, ultrasonic treatment. As the suspending medium, a suitable buffer solution (for example, phosphate buffer solution and the like) having pH value adjusted around neutral (pH 6.5 to 7.5) is preferably used. EDTA may be added to the solution for promoting crushing of cells. After crushing cells in this way, an insoluble component (inclusion body) is recovered by centrifugal separation or filtration according to any suitable method. To remove proteins derived from the prokaryotic cell as thoroughly as possible, washing with water, phosphate buffer solution, or the like is preferable. Washing with urea of about 4M is also permissible in some cases.

As the denaturing agent with which the resulting precipitation (pellet) is solubilized, a known denaturing agent, particularly, guanidine or urea can be used. The denaturing agent is used usually in the form of aqueous solution, and the concentration of the denaturing agent in the aqueous solution is, in case of guanidine, from 1 to 8 mol/liter, preferably from about 3 to 6 mol/liter, and in case of urea, from 5 to 9 mol/liter, preferably 8 mol/liter. Guanidine is usually used in the form of acid-added salt of guanidine, such as a guanidine hydrochloride and the like.

When a recombinant protein does not form an inclusion body in a prokaryotic host cell, a recombinant protein can be extracted by, after culturing, collecting the bacterial body by a method such as centrifugal separation and the like, and then solubilizing the cell using a denaturing agent, or by, after crushing, solubilizing the cells with a denaturing agent.

The denaturing agent used for solubilization of the collected cells includes, for example, guanidine and the like. The denaturing agent is used usually in the form of aqueous solution, and the concentration of the denaturing agent in the aqueous solution is, in case of guanidine, usually from 1 to 8 mol/liter, preferably from about 3 to 6 mol/liter. Guanidine is usually used in the form of acid-added salt of guanidine, such as a guanidine hydrochloride and the like.

Crushing of cells can be carried out by an ordinary method, for example, by ultrasonic treatment, French press and the like. As the denaturing agent used for solubilization of the crushed cells, a known denaturing agent, particularly, guanidine or urea can be used. The denaturing agent is used usually in the form of aqueous solution, and the concentration of the denaturing agent in the aqueous solution is, in case of guanidine, from 1 to 8 mol/liter, preferably from about 3 to 6 mol/liter, and in case of urea, from 5 to 9 mol/liter, preferably about 8 mol/liter. Guanidine is usually used in the form of acid-added salt of guanidine, such as a guanidine hydrochloride and the like.

Further, in general, when the recombinant protein expressed in a prokaryotic cell contains a cysteine residue, addition of a reducing agent on extraction using a denaturing agent is often conducted, whether an inclusion body is formed or not (Biotechnology and Bioengineering 413-13, 1993). This is conducted to cut an S-S bond in a recombinant protein, which is formed in a cell body or before the extraction (in may case, incorrect inter-molecular or intra-molecular S-S crosslinks, not present in a natural type), and for this purpose, it is usual to add the agent having strong reducing power such as DTT and the like at higher concentration (at least 10 mM or more). Therefore, to conduct a refolding operation accompaning formation of an S-S bond after extraction, it is necessary to remove a reducing agent by dialysis or gel filtration before the refolding.

In contrast, the production process of the present invention uses a weak reducing agent (having a reduction potential higher than -331 mV)(for example, 2-mercaptoethanol, cysteamine and the like) at a low concentration (from about 0.1 mM to about 50 mM, preferably from about 1 mM to about 10 mM) as an antioxidant in an extraction step in order to prevent formation of an S-S bond on extraction using a denaturing agent.

In the production process of the present invention, there is no necessity for removing the reducing agent before the refolding as in conventional technologies, since a weak reducing agent is used at low concentration.

As the reducing agent to be added to an extraction solution containing a denaturing agent in extracting a recombinant protein, any reducing agent can be used as long as it is useful for making the present invention, and it includes glutathione, cysteine, cysteamine and the like in addition to 2-mercaptoethanol. In terms of yield of a recombinant protein, the preferable concentration of the reducing agent is from 0.1 to 50 mmol/liter, particularly preferably from 1 to 10 mmol/liter.

After solubilizing an inclusion body, or solubilizing directly a cell body with a denaturing agent, or solubilizing crushed cells with a denaturing agent, under the condition as described above, impurities are removed by centrifugal separation and the like, and then refolding (activation, renaturation) of a recombinant protein can be carried out in the recovered supernatant.

The refolding is conducted by diluting about 10 to 25-fold the supernatant containing a recombinant protein with a buffer solution. In this case, it is desirable to effect dilution at neutral pH suitable for the protein activation until the concentration of denaturing agent reaches an ineffective concentration. When the denaturing agent is guanidine, it is desirable to effect dilution to the guanidine concentration of from 0 to 2.0 mol/liter, preferably about 1 mol/liter or less in the diluted solution, and when the denaturing agent is urea, it is desirable to effect dilution to the urea concentration of from 0 to 4.0 mol/liter, preferably about 2 mol/liter or less in the diluted solution.

In the production process of the present invention, the buffer solution for dilution used for refolding may contain an amino acid having no thiol group (mercapto group) (for example, arginine and the like).

As apparent from Example 5 described later, addition of an amino acid having no thiol group (for example, arginine and the like) gives, in combination with said addition of a low concentration of a reducing agent in extracting, a remarkable increase in yield of a recombinant protein or salt thereof, unexpectedly.

Further, a redox buffer (oxidized glutathione (GSSG) and reduced glutathione (GSH); cysteine and cystine; or cysteamine and cystamine; and the like) may also be added to the buffer solution for dilution in the refolding. The each concentration of an oxidizing agent and a reducing agent in the redox buffer is generally from 0.01 to 100 mmol/liter, particularly preferably from 0.1 to 10 mmol/liter.

As the amino acid having no thiol group added to the buffer solution for dilution in the refolding, any amino acid having no thiol group can be used as long as it is useful for making the present invention, and it includes aspartic acid, valine, lysine, alanine, citrulline and the like, in addition to arginine. In terms of yield of a recombinant protein in the refolding, the preferable concentration of the amino acid is from 0.1 to 2.0 mol/liter, particularly preferably from 0.1 to 1.0 mol/liter.

After solubilization and before refolding, known conventional purification processes, such as extraction, salting out, dialysis, partitioning, crystallization, recrystallization, gel filtration, chromatography and the like, can be carried out. Preferably, purification can be conducted, for example, by Sephadex G-25 (Pharmacia Biotech) in a 0.1 mol/liter phosphate buffer solution. Separation of the denaturing agent is also possible by dialysis against a 0.1 mol/liter phosphate buffer solution, in some cases.

The purification process can also be conducted after the refolding. In general, such a purification process includes, for example, extraction, salting out, dialysis, partitioning, crystallization, re-crystallization, gel filtration, chromatography and the like. Preferred examples are purification by dialysis, ion exchange chromatography through, for example, SP-Sepharose FF (Pharmacia Biotech), CM-5PW (Toso Co., Ltd.) or DEAE-5PW (Toso Co., Ltd.), reverse phase chromatography using for example ODP-50 (Showa Denko), and the like.

A recombinant protein obtained according to the present invention has the same activity as that of its natural protein already known, and can be used in the same manner as in the method of using the natural protein.

When bases and amino acids and the like are represented by abbreviations in the specification and drawings, they are based on abbreviations by IUPAC-IUB Commission on Biochemical Nomenclature or conventional abbreviations in this field, and examples thereof are described below. When an amino acid has optical isomers, it represents an L form unless otherwise stated.

| cDNA | complementary deoxyribonucleic acid |
|---|---|
| A | adenine |
| T | thymine |
| G | guanine |
| C | cytosine |
| RNA | ribonucleic acid |
| mRNA | messenger ribonucleic acid |
| EDTA | ethylene diamine tetraacetic acid |
| SDS | sodium dodecyl sulfate |
| 2-ME | 2-mercaptoethanol |
| DTT | dithiothreitol |
| Gly(G) | glycine |
| Ala(A) | alanine |
| Val(V) | valine |
| Leu(L) | leucine |
| Ile(I) | isoleucine |
| Ser(S) | serine |
| Thr(T) | threonine |
| Cys(C) | cysteine |
| Met(M) | methionine |
| Glu(E) | glutamic acid |
| Asp(D) | aspartic acid |
| Lys(K) | lysine |
| Arg(R) | arginine |
| His(H) | histidine |
| Phe(F) | phenylalanine |
| Tyr(Y) | tyrosine |
| Trp(W) | tryptophan |
| Pro(P) | proline |
| Asn(N) | asparagines |
| Gln(Q) | glutamine |
| Asx | Asp+Asn |
| Glx | Glu+Gln |

The SEQ ID NOs in the Sequence Listing of the present specification means the following sequences.
[SEQ ID NO: 1] This shows an amino acid sequence encoding human TL4.
[SEQ ID NO: 2] This shows a base sequence of a primer used in Reference Example 1 described later.
[SEQ ID NO: 3] This shows a base sequence of a primer used in Reference Example 1 described later.
[SEQ ID NO: 4] This shows an amino acid sequence coding mouse TL4.
[SEQ ID NO: 5] This shows a base sequence of a primer used in Reference Example 3 described later.
[SEQ ID NO: 6] This shows a base sequence of a primer used in Reference Example 3 described later.

The following reference examples and examples will illustrated the present invention more specifically, but do not limit the scope of the present invention.

### REFERENCE EXAMPLE

### Reference Example 1: Constructing a plasmid for expression of soluble human TL4 in Escherichia coli

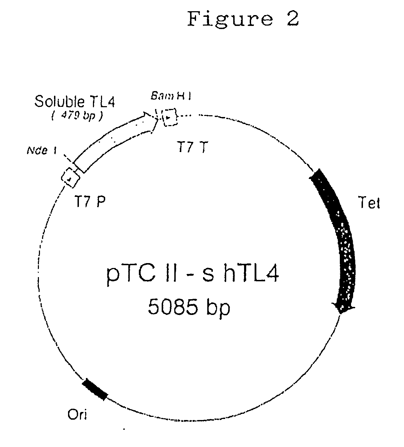

For obtaining a DNA fragment encoding from 84-th amino acid residue (Ile) to 240-th amino acid residue (Val) corresponding to an extracellular region of human TL4 (SEQ ID NO: 1), PCR (polymerase chain reaction) was conducted using a plasmid pTB1939 described in Reference Example 1 of JP-A No.11-141106 as a template, and two oligonucleotides 
(SEQ ID NO: 2 5'-TATACATATGATACAAGAGCGAAGGTC-3';
SEQ ID NO: 3 5'-AGCCGGATCCGACCTCACACCATGAAA-3')
 as primers. The resulting PCR product was subcloned with TA-system, and its base sequence was confirmed. Then, the clone was digested with NdeI and BamHI, and the intended DNA fragment was isolated by fractionation on 2.0% agarose gel electrophoresis. This NdeI-BamHI fragment was linked, by using a T4DNA ligase, to the downstream of T7 promoter in pTCII, which was also digested with NdeI and BamHI, to obtain a plasmid pTCII-shTL4 (Fig. 2).

### Reference Example 2: Expression of soluble human TL4 in Escherichia coli

Into *Escherichia coli* MM294 (DE3) having a T7 RNA polymerase gene (under lac promoter control) was introduced the plasmid pTCII-shTL4 obtained in the above-mentioned Reference Example 1, to obtain *Escherichia coli* MM294 (DE3)/pTCII-shTL4.

This transformed cell was cultured while shaking at 37°C for 8 hours in a 2-liter flask containing 1 liter of an LB medium (1% peptone, 0.5% yeast extract, 0.5% sodium chloride) with 10 µg/ml of tetracycline. The resulting culture was transplanted into a 50-liter fermentation bath containing 19 liters of a main fermentation medium (1.68% sodium monohydrogen phosphate, 0.3% potassium dihydrogen phosphate, 0.1% ammonium chloride, 0.05% sodium chloride, 0.05% magnesium sulfate, 0.02% defoaming agent, 0.00025% ferrous sulfate, 0.0005% thiamine hydrochloride, 1.5% glucose, 1.5% casamino acid) with 5µg/ml of tetracycline, and culturing was initiated while stirring under aeration at 37°C. When the turbidity of culture solution reached about 500 cret units, 75 µM of isopropyl-β-d-thiogalactopyranoside (IPTG) was added, and culturing was continued for further 2 hours (1480 cret units). Finally, this culture was subjected to centrifuge separation to obtain about 210 g of wet cell body, which were frozen and stored at -80°C.

The amount of soluble human TL4 expressed in the cell was estimated to about 4 mg/g wet cell body (50 mg/L) based on the staining intensity of a 17 Kd band representing the soluble human TL4 on SDS-PAGE of the cell extract.

The transformant *Escherichia coli* MM294 (DE3)/pTCII-shTL4 was deposited with Ministry of International Trade and Industry, Agency of Industrial Science and Technology, National Institute of Bioscience and Human-Technology (NIBH), Higashi 1-1-3, Tsukuba city, Ibaraki prefecture, Japan, under the deposition number of FERM BP-7019 from February 2, 2000, and deposited with Institute for Fermentation, Osaka (IFO), Juso Honmachi 2-17-85, Yodogawa ku, Osaka city, Osaka prefecture, Japan, on January 20, 2000 under the deposition number of IFO 16356.

The transformants *Escherichia coli* DH10B/pTB1939 and *Escherichia coli* DH10B/pTB1940, which contain DNA encoding human TL4 having an amino acid sequence represented by SEQ ID NO: 1 was deposited with Ministry of International Trade and Industry, Agency of Industrial Science and Technology, National Institute of Bioscience and Human-Technology (NIBH), Higashi 1-1-3, Tsukuba city, Ibaraki prefecture, Japan, under the deposition numbers of FERM BP-5595 and FERM BP-5596, respectively, from July 17, 1996, and deposited with Institute for Fermentation, Osaka (IFO), Juso Honmachi 2-17-85, Yodogawa ku, Osaka city, Osaka prefecture, Japan, from July 11, 1996 under the deposition numbers of IFO 15997 and IFO 15998, respectively.

### Reference Example 3: Constructing a plasmid for expression of soluble mouse TL4 in Escherichia coli

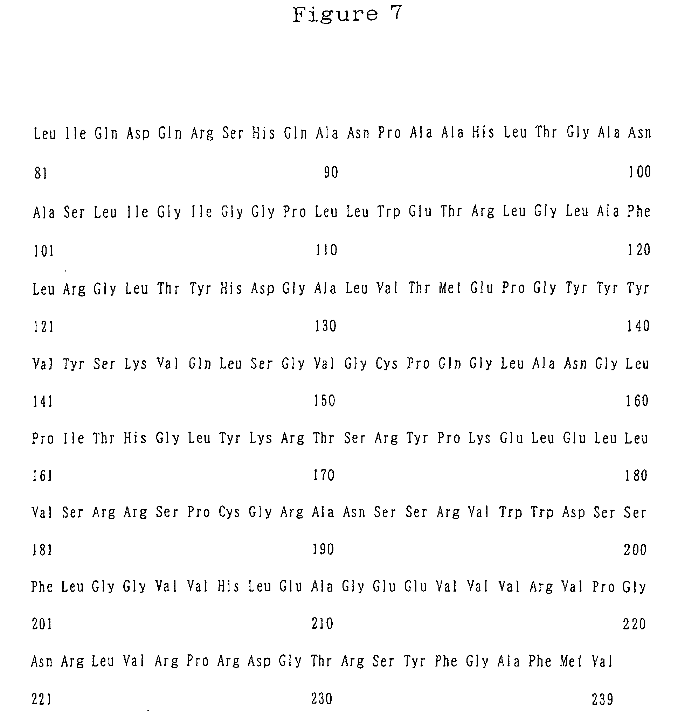

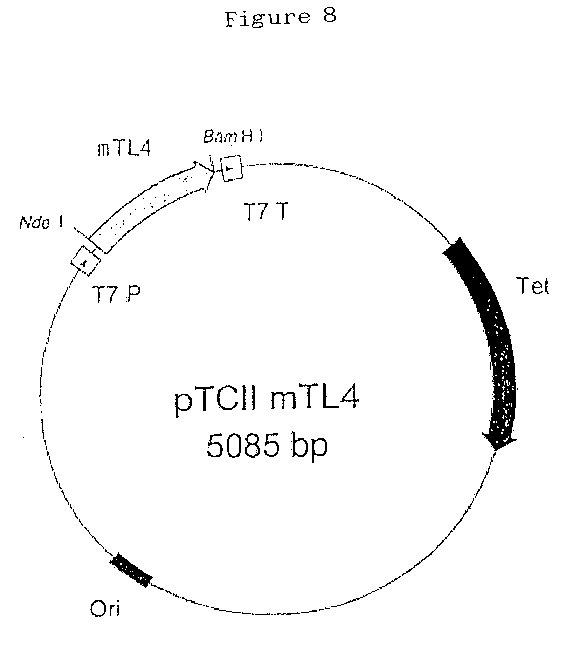

For obtaining a DNA fragment encoding from 81-th amino acid residue (Leu) to 239-th amino acid residue (Val) (Fig. 7), corresponding to an extracellular region, of the amino acid sequence encoding mouse TL4 (SEQ ID NO: 4), PCR (polymerase chain reaction) was conducted using a plasmid pTB1958 described in Reference Example 2 of WO 98/03648 as a template, and two oligonucleotides 
(SEQ ID NO: 5    5'-GGCATATGCTGATACAAGATCAACGATCTC-3';
SEQ ID NO: 6    5'-CGGATCCTCAGACCATGAAAGCTCCGAAAT-3')
 as primers. The resulting PCR product was subcloned with TA-system, and its base sequence was confirmed. Then, the clone was digested with NdeI and BamHI, and the intended DNA fragment was isolated by fractionation on 2.0% agarose gel electrophoresis. This NdeI-BamHI fragment was linked, by using a T4DNA ligase, to the downstream of T7 promoter in pTCII, which was also digested with NdeI and BamHI, to obtain a plasmid pTCII-mTL4 (Fig. 8).

### Reference Example 4: Expression of soluble mouse TL4 in Escherichia coli

Into *Escherichia coli* MM294 (DE3) having a T7 RNA polymerase gene (under lac promoter control) was introduced the plasmid pTCII-mTL4 obtained in the above-mentioned Reference Example 3, to obtain *Escherichia coli* MM294 (DE3)/pTCII-mTL4.

This transformed cell was cultured while shaking at 37°C for 8 hours in a 2-liter flask containing 1 liter of an LB medium (1% peptone, 0.5% yeast extract, 0.5% sodium chloride) with 10 µg/ml of tetracycline. The resulting culture was transplanted into a 50-liter fermentation bath containing 19 liters of a main fermentation medium (1.68% sodium monohydrogen phosphate, 0.3% potassium dihydrogen phosphate, 0.1% ammonium chloride, 0.05% sodium chloride, 0.05% magnesium sulfate, 0.02% defoaming agent, 0.00025% ferrous sulfate, 0.0005% thiamine hydrochloride, 1.5% glucose, 1.5% casamino acid) with 5µg/ml of tetracycline, and culturing was initiated while stirring under aeration at 37°C. When the turbidity of culture solution reached about 500 cret units, 75 µM of isopropyl-β-d-thiogalactopyranoside (IPTG) was added, and culturing was continued for further 4 hours (1905 cret units). Finally, this culture was subjected to centrifuge separation to obtain about 210 g of wet cell body, which were frozen and stored at -80°C.

The amount of soluble mouse TL4 expressed in the cell was estimated to about 50 mg/g wet cell body (550 mg/L) based on the staining intensity of a 17 Kd band representing the soluble mouse TL4 on SDS-PAGE of the cell extract.

The transformant *Escherichia coli* MM294 (DE3)/pTCII-TL4 was deposited with Ministry of International Trade and Industry, Agency of Industrial Science and Technology, National Institute of Bioscience and Human-Technology (NIBH), Higashi 1-1-3, Tsukuba city, Ibaraki prefecture, Japan, under the deposition number of FERM BP-7481 from March 5, 2001, and deposited with Institute for Fermentation, Osaka (IFO), Juso Honmachi 2-17-85, Yodogawa ku, Osaka city, Osaka prefecture, Japan, on February 1, 2001 under the deposition number of IFO 16550.

### EXAMPLES

### Example 1: Extraction of soluble human TL4 from Escherichia coli

To 25 g of the cells obtained in Reference Example 2 was added 125 mL of 50 mM Tris/HCl and 5 mM EDTA (pH 7.5). The cells were crushed at 4°C for 5 minutes repeatedly using an ultrasonic crusher (Sonifer 450)(Branson Ultrasonics Corporation), and then centrifugal separation (15000 rpm, 30 minutes) was conducted to obtain a precipitated fraction (inclusion body fraction). This precipitated fraction was washed with 500 ml of 50 mM Tris/HCl and 5 mM EDTA (pH 7.5), and with 50 ml of 50 mM Tris/HCl and 4 M urea (pH 7.5). Then, to the precipitated fraction was added 75 ml of 50 mM Tris/HCl, 4 M guanidine hydrochloride (pH 7.5) and 5 mM 2-mercaptoethanol, and the mixture was stirred at 4°C overnight, after which centrifugal separation (15000 rpm, 30 minutes) was conducted to obtain a supernatant.

### Example 2: Refolding of soluble human TL4

To the supernatant obtained in Example 1 was added 1.5 liter of 0.8 M arginine and 50 mM Tris/HCl (pH 8.0) and the mixture was incubated at 4 °C overnight for activation of proteins.

### Example 3: Purification of soluble human TL4

The solution containing renaturated proteins by the activation, obtained in Example 2, was adjusted to pH 6; condensed through an ultrafiltration membrane (regenerated cellulose membrane, fraction molecular weight 3 K (k dalton), membrane area 0.1 m² × 2)(Millipore Corporation); diluted 4-fold with distilled water; and subjected to centrifuge separation (8000 rpm, 15 minutes) to obtain a supernatant. After the supernatant was applied for adsorption on SP-Sepharose FF (1.1 cm ID × 5 cm L, 5 mL)(Pharmacia Biotech) equilibrated with 50 mM acetic acid buffer (pH 6.0) and 150 mM NaCl elution was made at a flow rate of 4 ml/min. for 40 minutes with a gradient of 0 to 60% B (B=50 mM acetic acid buffer (pH 6.0) and 1.5 M NaCl), and then a fraction containing TL4 was pooled. Subsequently, the fraction was applied for adsorption on CM-5PW (21.5 mm ID × 150 mm L, 13 µm)(Toso Co., Ltd.) equilibrated with 50 mM acetic acid buffer (pH 6.0) and 150 mM NaCl, and elution was made at a flow rate of 5 ml/min. for 30 minutes with a gradient of 0 to 40% B (B=50 mM acetic acid buffer (pH 6.0) and 1.5 M NaCl). A fraction containing TL4 was pooled, and diluted 2-fold with distilled water for preventing precipitation of TL4. This diluted solution was condensed through an ultrafiltration membrane (Amicon 8050 YM-10)(Millipore Corporation), and then substituted with 50 mM acetic acid buffer (pH 6.0) and 150 mM NaCl to obtain about 1.5 mg of soluble human TL4.

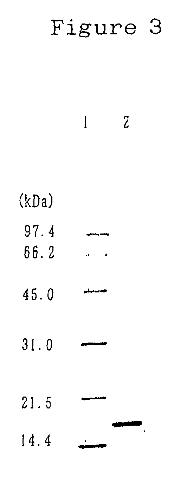

To determine the purity level of thus obtained soluble human TL4, SDS polyacrylamide gel electrophoresis was conducted. The product was suspended in Sample buffer [Laemmli, Nature, 227, 680 (1979)] containing 100 mM DTT, and heated at 95°C for 1 minute, then, electrophoresis was conducted on Multigel 15/25 (Daiichi Pure Chemicals Co., Ltd). Staining the gel with Coomassie brilliant blue after electrophoresis revealed a single band protein at about 17 Kd. This result shows that the product of soluble human TL4 is composed of the single component and has an extremely high purity (Fig. 3).

### Example 4: Analysis of soluble human TL4 using HPLC

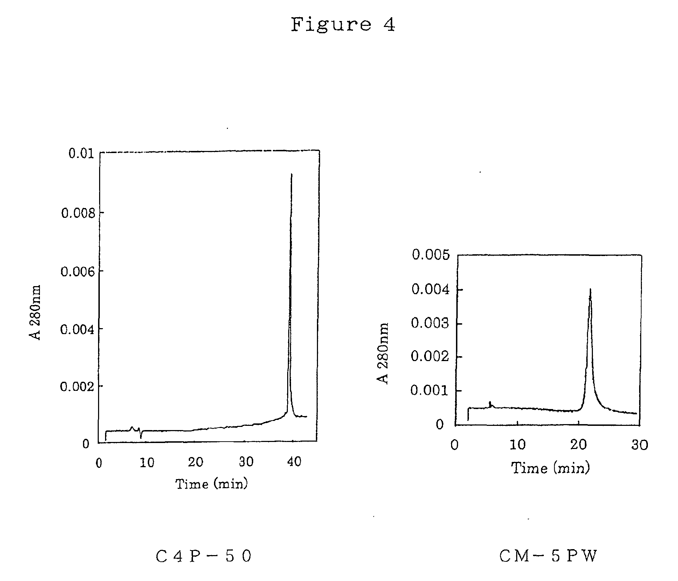

To determine the purity of soluble human TL4 obtained in Example 3, ion exchange HPLC and reverse phase HPLC were conducted using Gilson HPLC system (Gilson) for analysis. In the ion exchange HPLC, 5 µg of soluble human TL 4 was applied to CM-5PW (7.5 mm ID × 75 mm L, 10 µm)(Toso Co., Ltd.) equilibrated with 50 mM acetic acid buffer (pH 5.8) and 150 mM NaCl, and eluted at a flow rate of 0.8 ml/min. for 30 minutes at a gradient of 0 to 30% B (B=50 mM acetic acid buffer (pH 5.8), 1.5 M NaCI). In reverse phase HPLC, 2 µg of soluble human TL 4 was applied to C4P-50 (4.6 mm ID × 250 mm L, 5 µm)(Showa Denko K.K.) equilibrated with 30% B (A=0.1% trifluoroacetic acid (TFA), B=80% acetonitrile/0.1% TFA), and eluted at a flow rate of 0.5 ml/min. for 40 minutes with a gradient of 30 to 60%B. Detection was conducted at a wavelength of 280 nm, and the obtained data was subjected to waveform treatment by Chromato coder 21 (System Instruments) to calculate the purity. As a result, the soluble TL4 showed a single peak, indicating that the product of soluble human TL4 is composed of the single component and an extremely high purity (Fig. 4).

### Example 5: Effect on yield of soluble human TL4; Effect of addition of a reducing agent in extracting and effect of addition of amino acid in refolding

To 10 g of the cells obtained in Reference Example 2 was added 50 mL of 50 mM Tris/HCl (pH 7.5). The cells were crushed at 4°C for 5 minutes 4 times repeatedly using an ultrasonic crusher (Sonifer 450)(Branson Ultrasonics Corporation), and then centrifugal separation (15000 rpm, 30 minutes) was conducted to obtain a precipitated fraction (inclusion body fraction). This precipitated fraction was washed with 10 ml of 50 mM Tris/HCl and 5 mM EDTA (pH 7.5), and with 10 ml of 50 mM Tris/HCl and 3 M urea (pH 7.5). Then, to the precipitated fraction corresponding to 5 g of the cell was added 15 ml of 50 mM Tris/HCl, 4 M guanidine hydrochloride (pH 7.5) and 0 or 5 mM 2-mercaptoethanol, and the mixture was stirred at 4°C overnight, after which centrifugal separation (15000 rpm, 30 minutes) was conducted to obtain a supernatant.

To 1 ml of the supernatant was added 20 ml of a refolding solution of 50 mM Tris/HCl and 0, 0.2, 0.4, 0.6 or 0.8 M arginine (pH 8.0). After the mixture was incubated at 4 °C overnight for the protein activation, the mixture was adjusted to pH 6 with acetic acid, diluted 4-fold with distilled water, and subjected to centrifugal separation (3000 rpm, 15 minutes) to obtain a supernatant. The supernatant was applied for adsorption on SP-Sepharose FF (8 mm ID × 5 mm L)(Pharmacia Biotech) equilibrated with 50 mM acetic acid buffer (pH 6.0) and 150 mM NaCI, and elution was made at a flow rate of 1 ml/min. for 20 minutes with a gradient of 0 to 100% B (B=50 mM acetic acid buffer (pH 6.0), 1.5 M NaCl). After the obtained TL4 fraction was pooled, the amount of soluble human TL4 was quantified using C4P-50 (4.6 mm ID × 250 mm L, 5 µm)(Showa Denko) shown in Example 4.

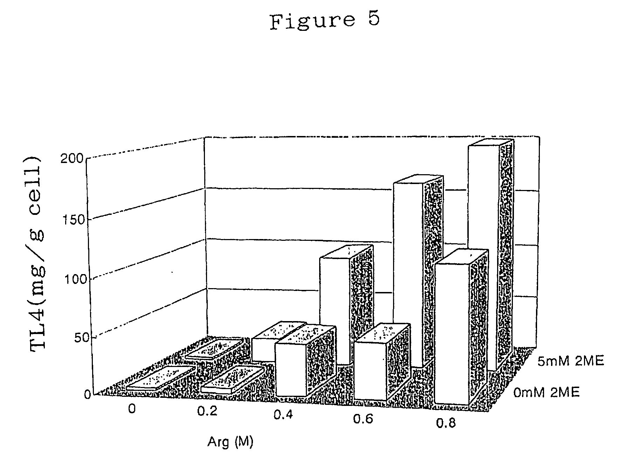

As a result, it was found that the yield of soluble human TL4 increases remarkably by addition of arginine in refolding (Fig. 5). Further, it was also found that the yield of soluble human TL4 increases more remarkably by synergistic action of combination of the addition of arginine and addition of 2-mercaptoethanol in extracting (Fig. 5). The optimal concentration of 2-mercaptoethanol in extracting was 5 mM, and the optimal concentration of arginine in refolding was 0.8 M in terms of the yield of soluble human TL4.

### Example 6: Protein chemical analysis of soluble human TL4

### (a) Amino acid composition analysis

Regarding soluble human TL4 obtained in Example 3, the amino acid composition thereof was determined by using an amino acid analyzer (Beckman System 6300E). As a result, the determined values of the product were identical to the theoretical values of an amino acid composition of soluble human TL4 to which Met was added to the N terminal (Table 1).

**Table 1**

| Amino acid | Number of residues per mol | Value anticipated from the base sequence of soluble human TL4 |
|---|---|---|
| Asx | 5.9 | 6 |
| Thr¹⁾ | 8.7 | 9 |
| Ser¹⁾ | 13.8 | 15 |
| Glx | 14.1 | 14 |
| Pro | 6.5 | 6 |
| Gly | 18.6 | 19 |
| Ala | 9.7 | 10 |
| Cys²⁾ | N.D. | 2 |
| Val | 12.7 | 15 |
| Met | 1.9 | 1 |
| Ile | 2.7 | 3 |
| Leu | 21 | 21 |
| Tyr | 7.9 | 8 |
| Phe | 3.8 | 4 |
| His | 5.0 | 5 |
| Lys | 12.0 | 12 |
| Arg | 7.1 | 7 |
| Trp | 2.6 | 3 |

| | | |
|---|---|---|
| Acid hydrolysis (average value resulted from hydrolysis for 24 and 48 hours at 110°C using 6 N HCl-4% thioglycolic acid) 1) Value was extrapolated at 0 hour. | | |
| 2) Undetected Analysis was conducted using about 10 µg of the product. | | |

### (b) Analysis of amino acid sequence at N-terminal

The amino acid sequence at N-terminal was determined using a gas phase protein sequencer (Applied Biosystem Model 492). The result was coincident with the N-terminal amino acid sequence of soluble human TL4, deduced from the base sequence, except that Met was added to the N-terminal of the obtained soluble human TL4 (Table 2).

**Table 2**

| Residue No. | PTH¹⁾-amino acid detected (pmol) | Amino acid expected from the base sequence of soluble human TL4 |
|---|---|---|
| 1 | Met(14) | |
| 2 | Ile(18) | Ile |
| 3 | Gln(9.8) | Gln |
| 4 | Glu(8.3) | Glu |
| 5 | Arg(7.1) | Arg |
| 6 | Arg(9.9) | Arg |
| 7 | Ser(3.6) | Ser |
| 8 | His(3.6) | His |
| 9 | Glu(3.6) | Glu |
| 10 | Val(3.8) | Val |

| | | |
|---|---|---|
| 1) phenylthiohydantoin Analysis was conducted using 50 pmol of the product. | | |

### (c) Analysis of amino acid at C-terminal

The C-terminal amino acid was determined using an amino acid analyzer (Beckman System 6300E). The result from soluble human TL4 was coincident with the C-terminal amino acid deduced from the base sequence (Table 3).

**Table 3**

| C-terminal amino acid | Recovery (%) |
|---|---|
| Val | 33.6 |
| Gas phase hydrazinolysis (100°C, 3.5 hours) Analysis was conducted using 4 nmol of the product. | |

### Example 7: Measuring activity of soluble human TL4

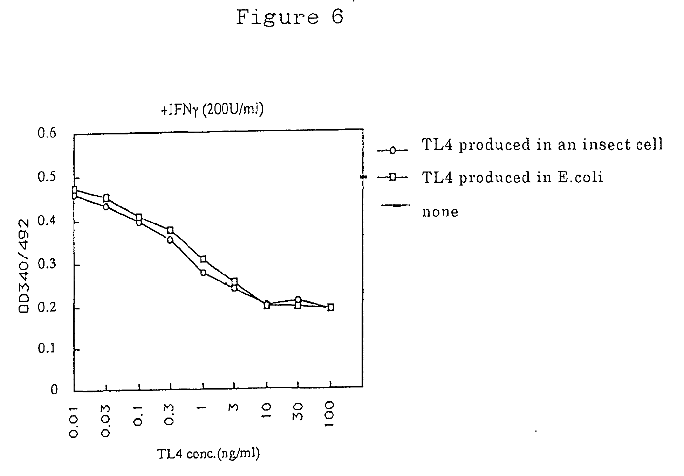

The purified soluble human TL4 obtained in Example 3 was examined for cytotoxicity on a clonal cancer cell. Cytotoxicity was measured as described below. A clonal human colon cancer cell WiDr was inoculated to a 96-well plate at 5000 cells/well, and soluble human TL4 produced in *Escherichia coli* or soluble human TL4 produced in an insect cell as described in Example 1 of JP-A No. 11-141106 was added thereto at various concentrations, in the absence or presence of interferon γ (Genzyme) at a final concentration of 200 U/ml. After 3 days of culturing, incorporation of bromodeoxyuridine was measured by Cell proliferation ELISA (Boehringer Co.,Ltd). As a result, in the presence of interferon γ, the soluble human TL4 produced in *Escherichia coli* showed excellent cytotoxicity, and its result was coincident with that of the soluble human TL4 produced in an insect cell (Fig. 6).

### Example 8: Extraction of soluble mouse TL4 from Escherichia coli

To 30 g of the cells obtained in Reference Example 4 was added 150 mL of 50 mM Tris/HCl and 5 mM EDTA (pH 7.5). The cells were crushed at 4°C for 5 minutes repeatedly using an ultrasonic crusher (Sonifer 450)(Branson Ultrasonics Corporation), and then centrifugal separation (15000 rpm, 30 minutes) was conducted to obtain a precipitated fraction (inclusion body fraction). This precipitated fraction was washed with 90 ml of 50 mM Tris/HCl and 5 mM EDTA (pH 7.5), and with 90 ml of 50 mM Tris/HCl and 4 M urea (pH 7.5). Then, to the precipitated fraction was added 900 ml of 100 mM Tris/HCl, 4 M guanidine hydrochloride (pH 7.5) and 5 mM cysteamine, and the mixture was stirred at 4°C overnight, after which centrifugal separation (8000 rpm, 30 minutes) was conducted to obtain a supernatant.

### Example 9: Refolding of soluble mouse TL4

To the supernatant obtained in Example 8 was added 22.5 liter of 0.8 M arginine and 50 mM Tris/HCl (pH 8.0) and the mixture was incubated at 4 °C overnight for activation of proteins.

### Example 10: Purification of soluble mouse TL4

The solution containing renaturated proteins by the activation, obtained in Example 9, was condensed through an ultrafiltration membrane (regenerated cellulose membrane, fraction molecular weight 10 K, membrane area 0.1 m² × 2)( Sartorius); adjusted to pH 6; diluted 4-fold with distilled water; and subjected to centrifuge separation (8000 rpm, 15 minutes) to obtain a supernatant. After the supernatant was applied for adsorption on SP-Sepharose FF (5 cm ID × 5 cm L, 100 mL)(Pharmacia Biotech) equilibrated with 50 mM acetic acid buffer (pH 6.0), TL4 was eluted by 50 mM acetic acid buffer (pH 6.0) and 0.75 M NaCl. Then, the fraction was applied for adsorption on SP-Sepharose HP (1.6 cm ID × 15 cm L, 25 µ)(Pharmacia Biotech) equilibrated with 50 mM acetic acid buffer (pH 6.0), and then elution was made at a flow rate of 4 ml/min. for 30 minutes with a gradient of 0 to 70% B (B=50 mM acetic acid buffer (pH 6.0), 1.5 M NaCl). Subsequently, the fraction was applied for adsorption on CM-5PW (21.5 mm ID × 150 mm L, 13 µm)(Toso Co., Ltd.) equilibrated with 50 mM acetic acid buffer (pH 6.0) and 150 mM NaCl, and elution was made at a flow rate of 5 ml/min. for 40 minutes with a gradient of 0 to 60% B (B=50 mM acetic acid buffer (pH 6.0) and 1.5 M NaCl). A fraction containing TL4 was pooled, and diluted 2-fold with distilled water for preventing precipitation of TL4. This diluted solution was condensed through an ultrafiltration membrane (Vivaspin 20, fraction molecular weight: 10 K)(Sartorius), and then substituted with 50 mM acetic acid buffer (pH 6.0) and 150 mM NaCl to obtain about 1.2 mg of soluble mouse TL4.

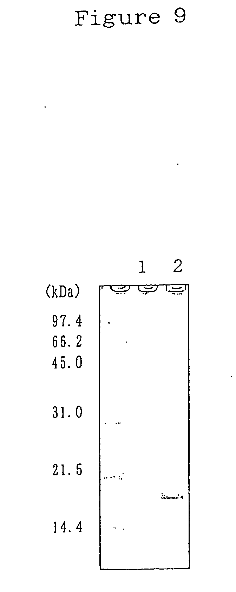

To determine the purity level of thus obtained soluble mouse TL4, SDS polyacrylamide gel electrophoresis was conducted. The product was suspended in Sample buffer [Laemmli, Nature, 227, 680 (1979)] containing 100 mM DTT, and heated at 95°C for 1 minute, then, electrophoresis was conducted on Multigel 15/25 (Daiichi Pure Chemicals Co., Ltd). Staining the gel with Coomassie brilliant blue after electrophoresis revealed a single band protein at about 17 Kd. This result shows that the product of soluble mouse TL4 is composed of the single component and has an extremely high purity (Fig. 9).

### Example 11: Analysis of soluble mouse TL4 using HPLC

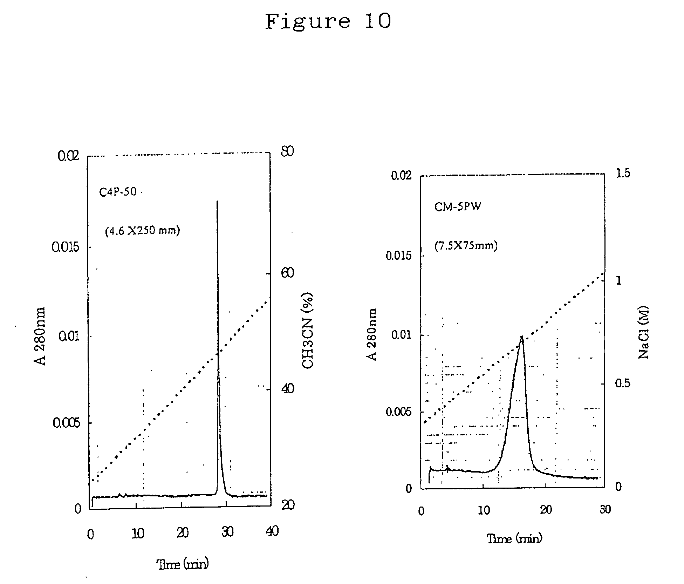

To determine the purity of soluble mouse TL4 obtained in Example 10, ion exchange HPLC and reverse phase HPLC were conducted using Gilson HPLC system (Gilson) for analysis. In the ion exchange HPLC, 10 µg of soluble mouse TL 4 was applied to CM-5PW (7.5 mm ID × 75 mm L, 10 µm)(Toso Co., Ltd.) equilibrated with 50 mM acetic acid buffer (pH 5.8) and 150 mM NaCl, and eluted at a flow rate of 0.8 ml/min. for 30 minutes at a gradient of 20 to 70% B (B=50 mM acetic acid buffer (pH 5.8), 1.5 M NaCl). In reverse phase HPLC, 2.5 µg of soluble mouse TL 4 was applied to C4P-50 (4.6 mm ID × 250 mm L, 5 µm)(Showa Denko K.K.) equilibrated with 30% B (A=0.1% trifluoroacetic acid (TFA), B=80% acetonitrile/0.1% TFA), and eluted at a flow rate of 0.5 ml/min. for 40 minutes with a gradient of 30 to 60%B. Detection was conducted at a wavelength of 280 nm, and the obtained data was subjected to waveform treatment by Chromato coder 21 (System Instruments) to calculate the purity. As a result, the soluble TL4 showed a single peak, indicating that the product of soluble mouse TL4 is composed of the single component and an extremely high purity (Fig. 10).

### Example 12: Effect on yield of soluble mouse TL4; Effect of addition of a reducing agent in extracting and effect of addition of amino acid in refolding

To 10 g of the cells obtained in Reference Example 4 was added 50 mL of 50 mM Tris/HCl and 5 mM EDTA (pH 7.5). The cells were crushed at 4°C for 5 minutes 4 times repeatedly using an ultrasonic crusher (Sonifer 450)(Branson Ultrasonics Corporation), and then centrifugal separation (15000 rpm, 30 minutes) was conducted to obtain a precipitated fraction (inclusion body fraction). This precipitated fraction was washed with 10 ml of 50 mM Tris/HCl and 5 mM EDTA (pH 7.5), and with 10 ml of 50 mM Tris/HCl and 4 M urea (pH 7.5). Then, to the precipitated fraction corresponding to 1 g of the cell was added 30 ml of 50 mM Tris/HCl, 4 M guanidine hydrochloride (pH 7.5) and 0 or 5 mM cysteamine, and the mixture was stirred at 4°C overnight, after which centrifugal separation (15000 rpm, 30 minutes) was conducted to obtain a supernatant.

To 0.8 ml of the supernatant was added 20 ml of a refolding solution of 50 mM Tris/HCl and 0, 0.2, 0.4, 0.6 or 0.8 M arginine (pH 8.0). After the mixture was incubated at 4 °C for 2 days for the protein activation, the mixture was adjusted to pH 6 with acetic acid, diluted 4-fold with distilled water, and subjected to centrifugal separation (3000 rpm, 15 minutes) to obtain a supernatant. In the supernatant, the amount of soluble mouse TL4 was quantified using C4P-50 (4.6 mm ID × 250 mm L, 5 µm)(Showa Denko) shown in Example 11.

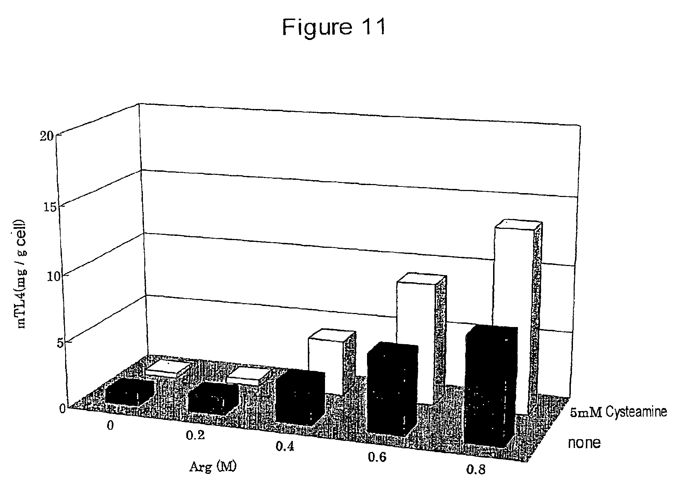

As a result, it was found that the yield of soluble mouse TL4 increases remarkably by addition of arginine in refolding (Fig. 11). Further, it was also found that the yield of soluble mouse TL4 increases more remarkably by synergistic action of combination of the addition of arginine and addition of cysteamine in extracting (Fig. 11). The optimal concentration of cysteamine in extracting was 5 mM, and the optimal concentration of arginine in refolding was 0.8 M in terms of the yield of soluble mouse TL4.

### Example 13: Protein chemical analysis of soluble human TL4

### (a) Amino acid composition analysis

Regarding soluble mouse TL4 obtained in Example 10, the amino acid composition thereof was determined by using an amino acid analyzer (Beckman System 6300E). As a result, the determined values of the product were identical to the theoretical values of an amino acid composition of soluble mouse TL4 to which Met was added to the N terminal (Table 4).

**Table 4**

| Amino acid | Number of residues per mol | Value anticipated from the base sequence of soluble mouse TL4 |
|---|---|---|
| Asx | 9.4 | 9 |
| Thr¹⁾ | 7.3 | 7 |
| Ser¹⁾ | 10.8 | 12 |
| Glx | 12.8 | 12 |
| Pro | 11.0 | 9 |
| Gly | 19.5 | 20 |
| Ala | 11.1 | 11 |
| Cys²⁾ | N.D. | 2 |
| Val | 13.2 | 14 |
| Met | 3.1 | 2 |
| Ile | 3.9 | 4 |
| Leu | 20 | 20 |
| Tyr | 7.8 | 8 |
| Phe | 4.1 | 4 |
| His | 4.9 | 5 |
| Lys | 3.3 | 3 |
| Arg | 13.4 | 14 |
| Trp | 2.6 | 3 |

| | | |
|---|---|---|
| Acid hydrolysis (average value resulted from hydrolysis for 24 and 48 hours at 110°C using 6 N HCl-4% thioglycolic acid) 1) Value was extrapolated at 0 hour. | | |
| 2) Undetected Analysis was conducted using about 10 µg of the product. | | |

### (b) Analysis of amino acid sequence at N-terminal

The amino acid sequence at N-terminal was determined using a gas phase protein sequencer (Applied Biosystem Model 492). The result was coincident with the N-terminal amino acid sequence of soluble mouse TL4, deduced from the base sequence, except that Met was added to the N-terminal of the obtained soluble human TL4 (Table 5).

**Table 5**

| Residue No. | PTH¹⁾-amino acid detected (pmol) | Amino acid expected from the base sequence of soluble human TL4 |
|---|---|---|
| 1 | Met(34) | |
| 2 | Leu(31) | Leu |
| 3 | Ile(35) | Ile |
| 4 | Gln(24) | Gln |
| 5 | Glu(20) | Glu |
| 6 | Gln(20) | Gln |
| 7 | Arg(15) | Arg |
| 8 | Ser(11) | Ser |
| 9 | His(6.4) | His |
| 10 | Gln(9.5) | Gln |

| | | |
|---|---|---|
| 1) phenylthiohydantoin Analysis was conducted using 100 pmol of the product. | | |

### INDUSTRIAL APPLICABILITY

According to the present invention, a recombinant protein in the biologically and pharmaceutically active form can be prepared in a large amount by efficiently changing an inactive form of the recombinant protein expressed in a prokaryotic cell using genetic engineering to the active form.

Among these active proteins to be thus obtained, for example, a Fas ligand-like protein TL4 is useful as an anti-cancer drug to treat cancers (breast carcinoma, prostate cancer, colon cancer, stomach cancer and the like), as an immunomodulator to treat cancers, virus infection, nephritis, autoimmune diseases, rheumatic arthritis and the like, and as a hepatic function modulator to treat hepatitis and the like.

## Claims

1. A method of producing a protein in the active form or salt thereof, comprising:
expressing a protein in a prokaryotic host cell by genetic engineering;
extracting the protein with a solution containing a reducing agent having a reduction potential of higher than -331 mV at a concentration of about 0.1 mM to about 50 mM; and
refolding the protein in a solution containing a mercapto-free amino acid or salt thereof.

2. The producing method according to claim 1, wherein the refolding is conducted in a solution containing (i) a mercapto-free amino acid or salt thereof, and (ii) reduced glutathione and oxidized glutathione, cysteine and cystine, or cysteamine and cystamine.

3. The producing method according to claim 1, wherein the protein is a Fas ligand-like protein.

4. The producing method according to claim 3, wherein the Fas ligand-like protein is TL4.

5. The producing method according to claim 1, wherein the reducing agent is a compound having a mercapto group.

6. The producing method according to claim 5, wherein the compound having a mercapto group is 2-mercaptoethanol or cysteamine.

7. The producing method according to claim 1, wherein the mercapto-free amino acid is arginine.

8. The producing method according to claim 1, comprising:
expressing a protein in a prokaryotic host cell by genetic engineering;
extracting and solubilizing the protein from the cell with a solution containing a reducing agent having a reduction potential of higher than -331 mV at a concentration of about 0.1 mM to about 50 mM, and a protein-denaturing agent; and
diluting the extract with a refolding solution containing a mercapto-free amino acid or salt thereof to reach an ineffective concentration of the denaturing agent.


[Sequence Listing]
<110> Takeda Chemical Industries, Ltd.
<120> Method for Producing of a Recombinant Protein
<130> 2707WOOP
<150> JP 12-97891
<151> 2000-03-30
<160> 6
<210> 1
<211> 240
<212> PRT
<213> Human
<400> 1
Met Glu Glu Ser Val Val Arg Pro Ser Val Phe Val Val Asp Gly Gln
1               5                   10                  15
Thr Asp Ile Pro Phe Thr Arg Leu Gly Arg Ser His Arg Arg Gln Ser
            20                  25                  30
Cys Ser Val Ala Arg Val Gly Leu Gly Leu Leu Leu Leu Leu Met Gly
        35                  40                  45
Ala Gly Leu Ala Val Gln Gly Trp Phe Leu Leu Gln Leu His Trp Arg
    50                  55                  60
Leu Gly Glu Met Val Thr Arg Leu Pro Asp Gly Pro Ala Gly Ser Trp
65                  70                  75                  80
Glu Gln Leu Ile Gln Glu Arg Arg Ser His Glu Val Asn Pro Ala Ala
                85                  90                  95
His Leu Thr Gly Ala Asn Ser Ser Leu Thr Gly Ser Gly Gly Pro Leu
            100                 105                 110

Leu Trp Glu Thr Gln Leu Gly Leu Ala Phe Leu Arg Gly Leu Ser Tyr
        115                 120                 125
His Asp Gly Ala Leu Val Val Thr Lys Ala Gly Tyr Tyr Tyr Ile Tyr
    130                 135                 140
Ser Lys Val Gln Leu Gly Gly Val Gly Cys Pro Leu Gly Leu Ala Ser
145                 150                 155                 160
Thr Ile Thr His Gly Leu Tyr Lys Arg Thr Pro Arg Tyr Pro Glu Glu
                165                 170                 175
Leu Glu Leu Leu Val Ser Gln Gln Ser Pro Cys Gly Arg Ala Thr Ser
            180                 185                 190
Ser Ser Arg Val Trp Trp Asp Ser Ser Phe Leu Gly Gly Val Val His
        195                 200                 205
Leu Glu Ala Gly Glu Lys Val Val Val Arg Val Leu Asp Glu Arg Leu
    210                 215                 220
Val Arg Leu Arg Asp Gly Thr Arg Ser Tyr Phe Gly Ala Phe Met Val
225                 230                 235                 240
<210> 2
<211> 27
<212> DNA
<213> Artificial Sequence
<220>
<223>
<400> 2
tatacatatg atacaagagc gaaggtc 27
<210> 3
<211> 27

<212> DNA
<213> Artificial Sequence
<220>
<223>
<400> 3
agccggatcc gacctcacac catgaaa          27
<210> 4
<211> 239
<212> PRT
<213> Mouse
<400> 4
Met Glu Ser Val Val Gln Pro Ser Val Phe Val Val Asp Gly Gln Thr
1               5                   10                  15
Asp Ile Pro Phe Arg Arg Leu Glu Gln Asn His Arg Arg Arg Arg Cys
            20                  25                  30
Gly Thr Val Gln Val Ser Leu Ala Leu Val Leu Leu Leu Gly Ala Gly
        35                  40                  45
Leu Ala Thr Gln Gly Trp Phe Leu Leu Arg Leu His Gln Arg Leu Gly
    50                  55                  60
Asp Ile Val Ala His Leu Pro Asp Gly Gly Lys Gly Ser Trp Glu Lys
65                  70                  75                  80
Leu Ile Gln Asp Gln Arg Ser His Gln Ala Asn Pro Ala Ala His Leu
                85                  90                  95
Thr Gly Ala Asn Ala Ser Leu Ile Gly Ile Gly Gly Pro Leu Leu Trp
            100                 105                 110
Glu Thr Arg Leu Gly Leu Ala Phe Leu Arg Gly Leu Thr Tyr His Asp

        115                 120                 125
Gly Ala Leu Val Thr Met Glu Pro Gly Tyr Tyr Tyr Val Tyr Ser Lys
    130                 135                 140
Val Gln Leu Ser Gly Val Gly Cys Pro Gln Gly Leu Ala Asn Gly Leu
145                 150                 155                 160
Pro Ile Thr His Gly Leu Tyr Lys Arg Thr Ser Arg Tyr Pro Lys Glu
                165                 170                 175
Leu Glu Leu Leu Val Ser Arg Arg Ser Pro Cys Gly Arg Ala Asn Ser
            180                 185                 190
Ser Arg Val Trp Trp Asp Ser Ser Phe Leu Gly Gly Val Val His Leu
        195                 200                 205
Glu Ala Gly Glu Glu Val Val Val Arg Val Pro Gly Asn Arg Leu Val
    210                 215                 220
Arg Pro Arg Asp Gly Thr Arg Ser Tyr Phe Gly Ala Phe Met Val
225 230 235 239
<210> 5
<211> 30
<212> DNA
<213> Artificial Sequence
<220>
<223>
<400> 5
ggcatatgct gatacaagat caacgatctc 30
<210> 6
<211>30
<212> DNA

<213> Artificial Sequence

<220>

<223>

<400> 6

cggatcctca gaccatgaaa gctccgaaat                                    30